# EUROPEAN PATENT APPLICATION

(11) **EP 4 066 796 A1**
(43) Date of publication of application: **05.10.2022**
(21) Application number: 21166407.3
(22) Date of filing: 31.03.2021
(51) Int. Cl.: A61F 5/11, A61K 8/81, A61Q 3/02

(54) **METHOD OF CORRECTING INGROWN FINGERNAIL OR TOENAIL**

(71) Applicant: Seon Jae, Lee, Incheon 102-2602 (KR); Yong Sun, Jang, Gyeonggi-do 101-1501 (KR); Taegsue, Kim, Seoul 55-304 (KR)
(72) Inventor: Seon Jae, Lee, 102-2602 Incheon (KR); Yong Sun, Jang, 101-1501 Gyeonggi-do (KR)
(74) Representative: Eder, Michael

(57) **Abstract**

One aspect of the present disclosure relates to a method of correcting an ingrown fingernail or toenail, and more particularly, to a method of correcting an ingrown fingernail or toenail to correct the ingrown fingernail or toenail using a force when the adhesive composition is upwardly curved and cured, by applying an adhesive composition and irradiating ultraviolet and visible rays on the ingrown fingernail or toenail.

## Description

### BACKGROUND OF THE DISCLOSURE

### 1. Field of the Disclosure

One aspect of the present disclosure relates to a method of correcting an ingrown fingernail or toenail, and more particularly, to a method of correcting an ingrown fingernail or toenail to correct the ingrown fingernail or toenail using a force when the adhesive composition is upwardly curved and cured, by applying an adhesive composition and irradiating ultraviolet and visible rays on the ingrown fingernail or toenail.

### 2. Description of the Related Art

An ingrown fingernail or toenail refers to a disease mainly occurring on the big toenail, in which the fingernail or toenail penetrates into the flesh, thereby causing inflammation and pain.

The ingrown fingernail or toenail may be caused by any situation in which the fingernail or toenail constantly presses the outer flesh. For example, the ingrown nail occurs occasionally due to an increase in internal pressure when a part of uncut fingernail or toenail penetrates into the flesh after cutting an outer side of the fingernail or toenail deeply using a nail clipper, when a shape of the fingernail or toenail is deformed due to a long period of neglect of the athlete's foot on the fingernail or toenail, when tight shoes are worn for a long time, when the toe bone protrudes, and when the fingernail or toenail has a naturally severe flection as obesity or aging progresses. In addition, when some family members have the ingrown fingernail or toenail, another family member also may have the ingrown fingernail or toenail, so it is believed that genetic factors are also involved.

The ingrown fingernail or toenail most often occurs in the big toe, especially in the thumb of the right foot. This is because the right toe is the area to which a pressure is applied most during walking or running. In the early stage of the ingrown fingernail or toenail, an outer or inner side of the finger or toe has mild pain while becoming slightly reddish and swelling. Soon, as the friction intensifies, the finger swells more and weeps, and the skin around the fingernail or toenail begins to fester. When the ingrown fingernail or toenail continues to progress, the odor is also generated. The case of ingrown toenail causes the difficulty in walking normally due to severe pain.

A preventive approach for the ingrown fingernail or toenail includes avoiding cutting the fingernail or toenail too short, and refraining from applying a pressure to the hand or foot. However, since the preventive approach has a limit with respect to the ingrown fingernail or toenail that have already occurred, methods for correcting the already ingrown fingernail or toenail have been proposed.

As a method for correcting the ingrown fingernail or toenail, a device such as the ingrowing toenail corrective device disclosed in Korean Patent Registration No. 10-1565692 may be used. The conventional ingrowing toenail corrective device is a device for properly correcting an ingrown toenail to a normal toenail by installing an elastic leaf spring to the toenail.

However, in order to correct the ingrown fingernail or toenail by wearing the conventional ingrown toenail corrective device, the corrective device is required to be worn constantly, thereby causing discomfort to a user performing external activities while wearing shoes or the like. In addition, the problem due to costs for purchasing the ingrown toenail corrective device may occur.

Accordingly, the need for a method capable of easily correcting the ingrown fingernail or toenail without a separate corrective device tends to increase.

### (Patent Document)

(Patent Document 1) Korean Patent Registration No. 10-1565692 (published on November 04, 2015) titled by "INGROWING TOENAIL CORRECTIVE DEVICE"

### SUMMARY OF THE DISCLOSURE

In order to solve the problems as the above, one aspect of the present disclosure provides a method of correcting an ingrown fingernail or toenail, in which an adhesive composition is applied and ultraviolet and visible rays are irradiated on the ingrown fingernail or toenail to curve both side portions of the ingrown fingernail or toenail upward using a force of the adhesive composition upon being upwardly curved and cured, thereby outwardly protruding both end portions penetrating into an inner side of a skin so as to correct the ingrown fingernail or toenail, so that additional correction tool is unnecessary, and thus the inconvenience is minimized and the correction cos is reduced.

In order to solve the above problems, one aspect of the present disclosure provides a method of correcting an ingrown fingernail or toenail, which includes: applying an adhesive composition on a top portion of an ingrown fingernail or toenail (S10); and irradiating ultraviolet and visible rays on the adhesive composition applied in the adhesive composition application step (S10) to allow both ends penetrating into an inner side of a skin of the ingrown fingernail or toenail to protrude outward (S20).

In addition, one aspect of the present disclosure may further include a step of attaching a protective base composition or protective tape on the ingrown fingernail or toenail, before performing step S10 (S5).

In addition, one aspect of the present disclosure may further include a step of attaching a protective coating composition or coating tape on a top portion of the adhesive composition, after performing step S20 (S25).

In addition, step S10 and step S20 may be repeatedly performed one to five times.

In addition, a surface of the adhesive composition of step S10 may be upwardly curved and cured when irradiated with ultraviolet or visible rays.

In the method of correcting an ingrown fingernail or toenail according to one aspect of the present disclosure, the adhesive composition is applied and the ultraviolet and visible rays are irradiated on the ingrown fingernail or toenail to curve the both side portions of the ingrown fingernail or toenail upward using the force of the adhesive composition upon being upwardly curved and cured, thereby outwardly protruding the both end portions penetrating into the inner side of the skin so as to correct the ingrown fingernail or toenail, so that additional correction tool is unnecessary, and thus the inconvenience can be minimized and the correction cost can be reduced.

### BRIEF DESCRIPTION OF THE DRAWINGS

FIG. 1 is a perspective view showing an appearance of a general ingrown fingernail or toenail.
FIG. 2 is an exemplary view showing a state in which a protective base composition is attached to an ingrown fingernail or toenail.
FIG. 3 is an exemplary view showing a state in which an adhesive composition is applied to the top portion of the ingrown fingernail or toenail.
FIG. 4 is an exemplary view showing an appearance in which ultraviolet and visible rays are irradiated to the adhesive composition on the top portion of the ingrown fingernail or toenail to curl the ingrown fingernail or toenail upwards, so that both end portions penetrating into an inner side of a skin protrude outward.
FIG. 5 is an exemplary view showing a state in which a protective coating composition is attached to the ingrown fingernail or toenail.
FIG. 6 is an exemplary view showing a state in which both side end portions of the ingrown fingernail or toenail protruding to the outside are growing.
FIGS. 7 to 10 are exemplary views showing a method of correcting the ingrown fingernail or toenail according to another embodiment of one aspect of the present disclosure.
FIG. 11 is an exemplary view showing the method of correcting the ingrown fingernail or toenail according to one aspect of the present disclosure so as to be easily understood.
FIG. 12 is an exemplary view showing the method of correcting the ingrown fingernail or toenail according to one aspect of the present disclosure so as to be easily understood.

### DETAILED DESCRIPTION OF THE DISCLOSURE

Hereinafter, the detailed descriptions of one aspect of the present disclosure are embodiments for carrying out one aspect of the present disclosure, and the corresponding embodiment refers to the accompanying drawings as an example. The embodiments will be described in detail to enable those skilled in the art to carry out one aspect of the present disclosure. It is apparent to be understood that the various embodiments of one aspect of the present disclosure may be different from each other but do not need to be mutually exclusive. For example, the particular shape, structure, and feature described herein may be embodied in other embodiments without departing from the idea and scope of the present disclosure in connection with the embodiment. In addition, it may be understood that the location or arrangement of an individual element within each disclosed embodiment may be modified without departing from the idea and scope of the present disclosure.

Accordingly, the following detailed description does not disclose a limited meaning, and the scope of the present disclosure is limited only by the appended claims, along with the full scope of equivalents to which the claims are entitled, if properly explained. Similar reference numerals in the drawings refer to the same or similar function throughout several aspects.

General term which is widely used recently has been selected in one aspect of the present disclosure in consideration of the function according to one aspect of the present disclosure as possible, however, the term may vary depending on the intention of those skilled in the art, judicial cases, the advent of new technology, or the like. Further, in certain cases, the term may be arbitrarily selected by the applicant, and in this case, the meaning thereof will be described in detail in the relevant description of the disclosure. Therefore, the term used in one aspect of the present disclosure may be defined based on the meaning of the term and contents throughout one aspect of the present disclosure, not simply on the name of the term.

When one part "includes" one element in one aspect of the present disclosure, the above expression does not exclude other elements, but may further include the other elements, unless particularly stated otherwise.

Hereinafter, a method of correcting an ingrown fingernail or toenail according to one aspect of the present disclosure will be described in detail with reference to the accompanying drawings.

The method of correcting an ingrown fingernail or toenail according to one aspect of the present disclosure includes: applying an adhesive composition 20 on a top portion of an ingrown fingernail or toenail N (S10); and irradiating ultraviolet and visible rays on the adhesive composition 20 applied in the adhesive composition application step S10 to allow both ends O1 and O2 penetrating into an inner side of a skin of the ingrown fingernail or toenail N to protrude outward (S20).

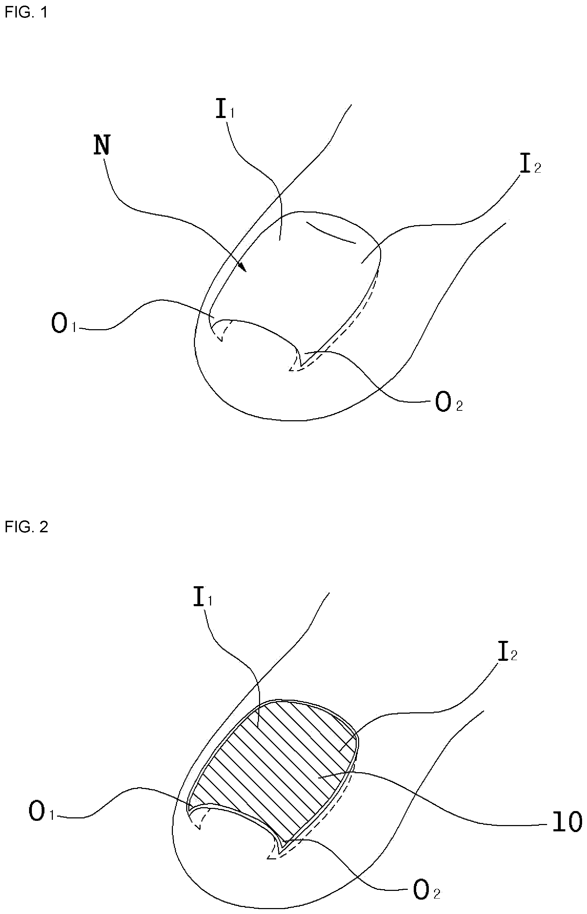

FIG. 1 is a perspective view showing an appearance of a general ingrown fingernail or toenail N. In the following description, when viewed from a fingertip while the fingernail or toenail faces upward, the left starting point from which the ingrown fingernail or toenail N starts is referred to as a left start portion 11, and the right starting point from which the ingrown fingernail or toenail N starts is referred to as a right start portion 12. In addition, when viewed from the fingertip while the fingernail or toenail faces upward, the left end point at which the ingrown fingernail or toenail N ends is referred to as a left portion O1, and the right point at which the ingrown fingernail or toenail N ends is referred to as a right portion 02.

As shown in FIG. 1, the general ingrown fingernail or toenail N is positioned in a state where the left end portion O1 and the right end portion O2 penetrate into the inside of the skin, thereby causing the corresponding site to be festered or inflamed. For this reason, the ingrown fingernail or toenail N may be corrected by outwardly protruding and fixing both side end portions O1 and O2 of the ingrown fingernail or toenail N.

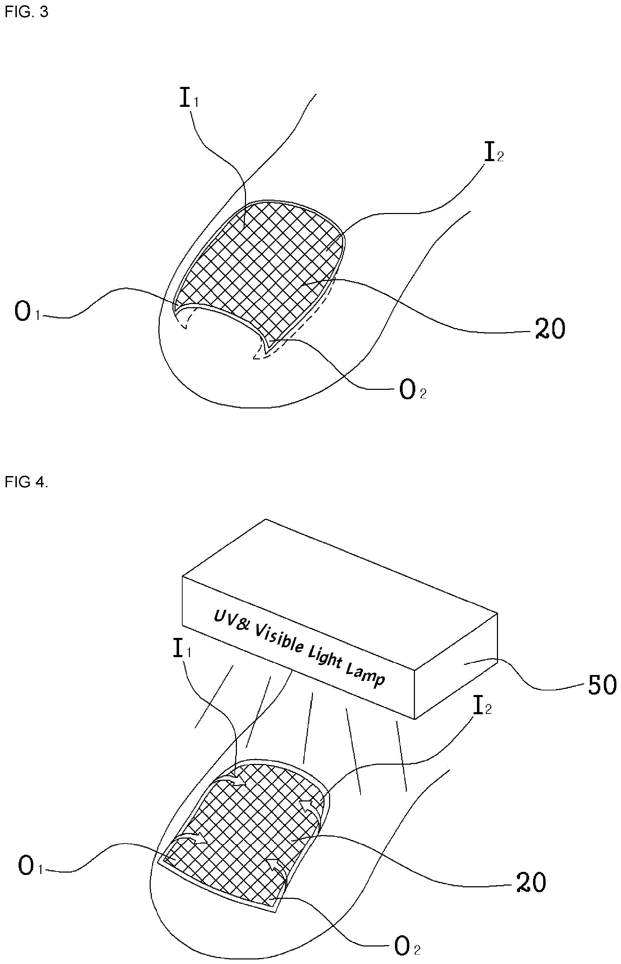

In step S10, as shown in FIG. 3, the adhesive composition 20 is applied on the top portion of the ingrown fingernail or toenail N.

In step S10, the top portion of the ingrown fingernail or toenail N may refer to a surface of the ingrown fingernail or toenail N, and may refer to the upper portion after the protective base composition, the protective tape or the like is adhered to the ingrown fingernail or toenail N. To summarize this, the top portion of the ingrown fingernail or toenail N includes the entire upper space of the ingrown fingernail or toenail N.

The adhesive composition 20 in step S10 may include suitable UV and visible ray-curable resin. The adhesive composition 20 may further include an additional composition in addition to the UV and visible ray-curable resin.

As an example, the adhesive composition 20 of step S10 may be a composition including at least one selected from urethane acrylates, epoxy acrylates, polyester acrylates, or acrylic acrylates. In addition, any adhesive composition may be included in the adhesive composition 20 of the present disclosure as long as it has a property of being cured through UV and visible rays. That is, the adhesive composition 20 may further include additional components in addition to the above components.

It is preferable that the adhesive composition 20 in step S10 is stored away from fire, and stored in a place out of reach of infants and children. In addition, the adhesive composition 20 is required to be used exclusively for fingernails or toenails, and is not recommended to be used on a site with abnormalities such as eczema and dermatitis. The adhesive composition 20 is required not to be used with soaps or anionic detergents. When the skin has an abnormal symptom, it may be appropriate to stop using the adhesive composition and consult a specialist.

In step S20, as shown in FIG. 4, the ultraviolet and visible rays are irradiated on the adhesive composition 20 applied in the adhesive composition application step (S10) to allow both side end portions O1 and O2 penetrating into an inner side of a skin of the ingrown fingernail or toenail N to protrude outward.

The adhesive composition 20 in step S20 has a property in which the surface is upwardly curved and cured when irradiated with the ultraviolet and visible rays.

In step S20, it is preferable that the adhesive composition 20 is irradiated with ultraviolet and visible rays using an ultraviolet and visible ray lamp 50. The ultraviolet and visible ray lamp 50 refers to a lamp having short wavelength, long wavelength, and visible rays used for sterilizing, disinfecting, and curing products, and may use a lamp that irradiates ultraviolet and visible rays with a light wavelength of 1 nm to 1,400 nm of UVV to UVC, that is, UVV, UVA, UVB, UVC, and the like. The adhesive composition 20 of one aspect of the present disclosure has a property in which the surface is upwardly curved and cured when the ultraviolet and visible rays are irradiated from the ultraviolet and visible ray lamp 50. As the surface is curved upward while the adhesive composition 20 is cured, that is, as the surface of the fingernail or toenail is curved and cured upward from the edge toward a center thereof, both side portions of the ingrown fingernail or toenail N is also curved upward by the force caused when the adhesive composition 20 is upwardly curved and cured, so that both side end portions O1 and O2 buried in the skin are exposed to the outside.

It is noted that the adhesive composition 20 is required not be applied to the skin around the ingrown fingernail or toenail N, and it is required to pay attention in step S20 since the user may feel slightly hot due to the ultraviolet and visible ray lamp 50 according to individual differences. Accordingly, in step S20, it may be preferably to irradiate the ultraviolet and visible rays with the ultraviolet and visible ray lamp 50 for 1 seconds to 90 seconds, and it may be more preferable to irradiate the ultraviolet and visible rays for 30 seconds to 60 seconds.

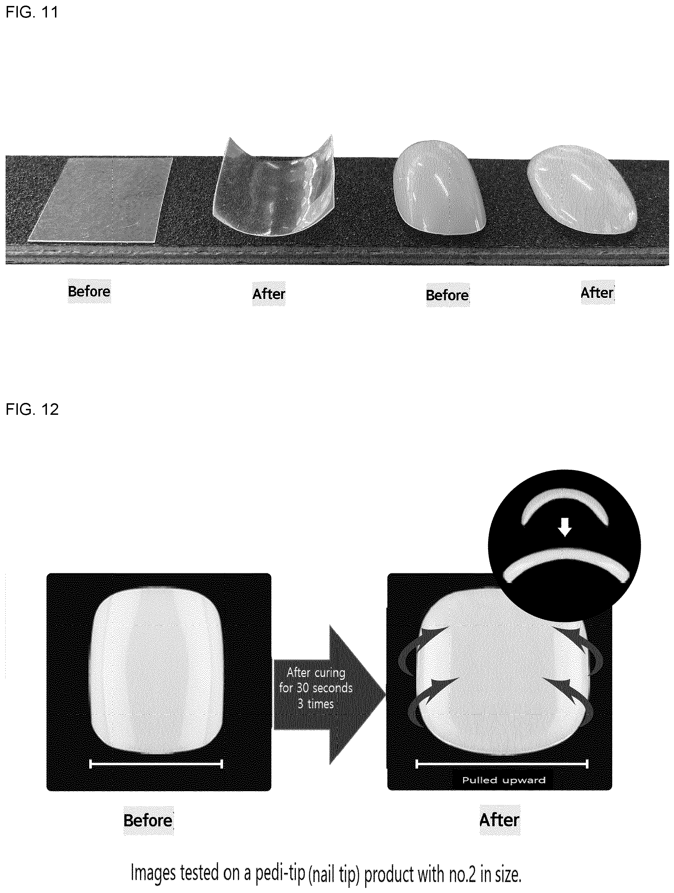

Meanwhile, FIG. 11 is an exemplary view showing the method of correcting the ingrown fingernail or toenail according to one aspect of the present disclosure so as to be easily understood. The plate disposed first from the left is a hard plate with no treatment. When the adhesive composition 20 is applied and the ultraviolet and visible rays are irradiated to the hard plate, it can be seen that both side portions are curved upward like the second plate from the left. In addition, the third fingernail or toenail from the left is a fingernail or toenail for nail art. When the adhesive composition 20 is applied to and the ultraviolet and visible rays are irradiated to the fingernail and toenail for nail art, it can be seen that both side portions are curved upward and opened to the left and right sides like the fourth fingernail or toenail from the left. In addition, FIG. 12 shows a state in which both side portions of the fingernail or toenail for nail art are curved upward after applying an adhesive composition 20 and irradiating ultraviolet and visible rays on the ingrown fingernail or toenail for nail art.

Meanwhile, according to the embodiments of one aspect of the present disclosure, steps S10 to S20 may be repeated 1 to 5 times. There may be variations depending on the individual, however, the both side end portions O1 and O2 of the ingrown fingernail or toenail N may be exposed to the outside by repeating the process of curing the adhesive composition 20 through the ultraviolet and visible rays while applying the adhesive composition 20 approximately 1 to 5 times. Most appropriately, three repetitions may be effective.

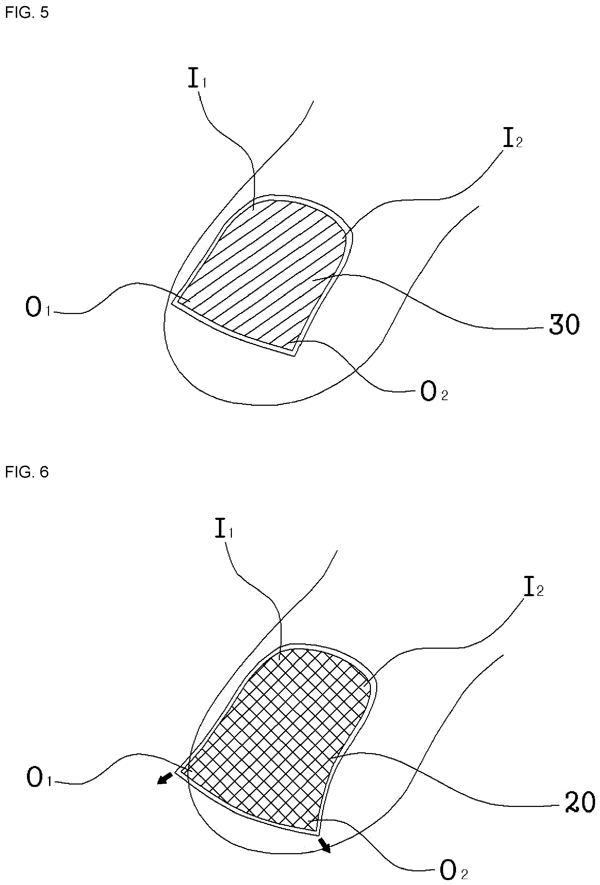

As in the above-described manner, the adhesive composition 20 is kept remain as it is on the ingrown fingernail or toenail N having the both side end portions O1 and O2, which have been buried in the skin, exposed to the outside, so that the fingernail or toenail grows in the above state as shown in FIG. 6. When the above treatment is repeated 1 to 5 times a month, and performed for about 2 months, the ingrown fingernail or toenail N may be fixed without further penetrating into the skin.

Meanwhile, according to the embodiments of one aspect of the present disclosure, the method may further include a step of attaching a protective base composition 10 or protective tape 10' on the ingrown fingernail or toenail, before performing the step S10, as shown in FIG. 2 (S5).

In addition, step S5 may further include a step of irradiating ultraviolet and visible rays to the protective base composition 10 or the protective tape 10' after the attaching of the protective base composition 10 or the protective tape 10'.

In step S5, the protective base composition 10 and the protective tape 10' serve to increase the adhesion between the ingrown fingernail or toenail N and the adhesive composition 20. It is appropriate that the protective base composition 10 and the protective tape 10' may have high adhesion to the ingrown fingernail or toenail N, and at the same time, it is appropriate to have high adhesion to the adhesive composition 20.

The protective base composition 10 in step S5 may include UV and visible ray curable resin. In addition, the protective base composition 10 may further include natural curable and solvent curable resin components in addition to the UV and visible ray curable resin.

As an example, the protective base composition 10 of step S5 may be a composition including one selected from urethane acrylates, epoxy acrylates, polyester acrylates, or acrylic acrylates. In addition, any protective base composition 10 may be included in the adhesive composition of the present disclosure as long as it has a property of being cured through UV and visible rays. That is, the protective base composition 10 may further include additional components in addition to the above components.

In addition, according to the embodiments of one aspect of the present disclosure, the method may further include a step of attaching a protective coating composition 30 or coating tape 30' on a top portion of the adhesive composition, after performing the step S20, as shown in FIG. 5 (S25).

In addition, step S25 may further include a step of irradiating ultraviolet and visible rays to the protective coating composition 30 or coating tape 30', after the attaching of the protective coating composition 30 or coating tape 30'.

In step S25, the protective coating composition 30 and the coating tape 30' serves to coat the adhesive composition 20 to increase a sustaining strength. The protective coating composition 30 and the coating tape 30' may prevent the adhesive composition 20 from being corroded to block external moisture or air, and may prevent an occurrence of cracks in the cured adhesive composition 20.

The protective coating composition 30 in step S25 may include UV and visible ray curable resin. In addition, the protective coating composition 30 may further include natural curable and solvent curable resin components in addition to the UV and visible ray curable resin.

As an example, the protective coating composition 30 of step S25 may be a composition including one selected from urethane acrylates, epoxy acrylates, polyester acrylates, or acrylic acrylates. In addition, any protective coating composition 30 may be included in the adhesive composition of the present disclosure as long as it has a property of being cured through UV and visible rays. That is, the protective coating composition 30 may further include additional components in addition to the above components.

Hereinafter, a method of applying the adhesive composition 20 according to various embodiments of one aspect of the present disclosure will be described.

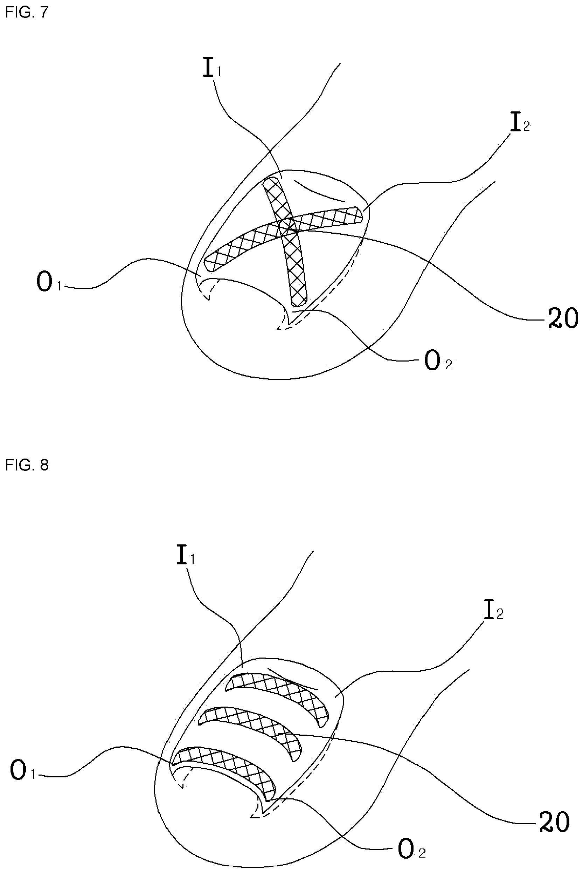

FIGS. 7 to 10 are exemplary views showing a method of correcting the ingrown fingernail or toenail according to another embodiment of one aspect of the present disclosure.

For the method of correcting the ingrown fingernail or toenail according to one aspect of the present disclosure, in step S10, the adhesive composition 20 may be applied to the top portion of the ingrown fingernail or toenail N while intersecting in diagonal directions as shown in FIG. 7.

In other words, the adhesive composition 20 may be applied while starting from the left start portion I1 of the ingrown fingernail or toenail N and then applied toward the right end portion O2, and may be applied while starting from the right start portion I2 of the ingrown fingernail or toenail N and then applied toward the left end portion O1.

When the adhesive composition 20 is applied crosswise in the diagonal directions as described in the above manner, the ingrown fingernail or toenail N having the deeply penetrating both side end portions O1 and O2 may be effectively corrected.

In addition, for the method of correcting the ingrown fingernail or toenail according to one aspect of the present disclosure, in step S10 the adhesive composition 20 may be applied to the top portion of the ingrown fingernail or toenail N in the form of a plurality of lines parallel to each other and orthogonal to the lengthwise direction of the finger as shown in FIG. 8.

In other words, the adhesive composition 20 may be applied in a row along the line connecting the left end portion O1 to the right end portion O2 of the ingrown fingernail or toenail N, and may be applied in at least one row parallel to the above line.

The case that the adhesive composition 20 is applied in the form of the lines parallel to each other and orthogonal to the lengthwise direction of the finger as in the above manner may be effective when the both side portions of the ingrown fingernail or toenail N deeply penetrate into the finger or toe.

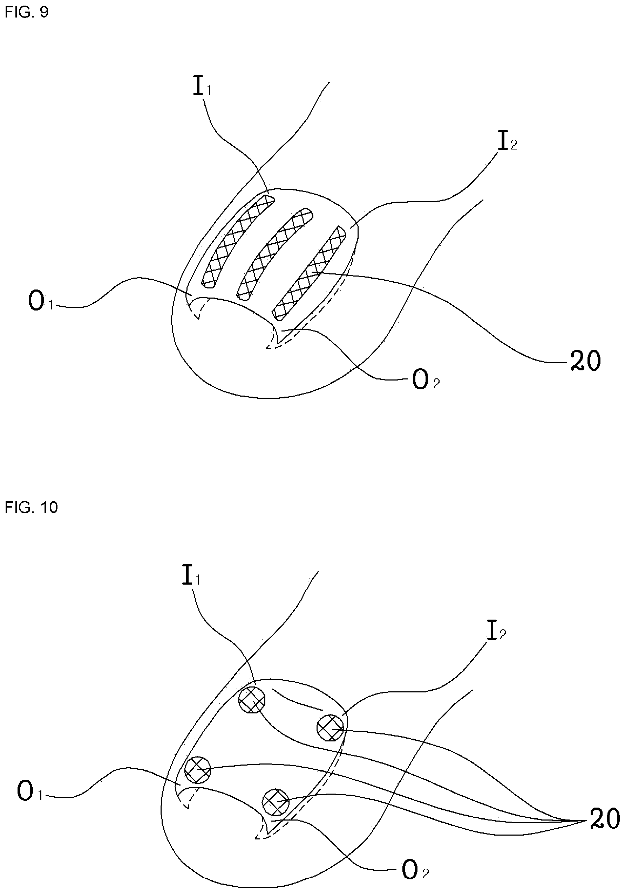

In addition, for the method of correcting the ingrown fingernail or toenail according to one aspect of the present disclosure, in step S10 the adhesive composition 20 may be applied to the top portion of the ingrown fingernail or toenail N in the form of a plurality of lines parallel to each other and matching with the lengthwise direction of the finger as shown in FIG. 9.

In other words, the adhesive composition 20 may be applied while starting from the left start portion I1 of the ingrown fingernail or toenail N, and then applied toward the left end portion O1, and may be applied while starting from the right start portion I2 of the ingrown fingernail or toenail N and then applied toward the right end portion O2.

The case that the adhesive composition 20 is applied in the form of the lines parallel to each other and matching with the lengthwise direction of the finger as in the above manner may be effective when the ingrown fingernail or toenail N is curved forward and penetrates into the finger or toe.

In addition, for the method of correcting the ingrown fingernail or toenail according to one aspect of the present disclosure, in step S10, the adhesive composition 20 may be applied to each corner of the ingrown fingernail or toenail N as shown in FIG. 10.

In other words, the adhesive composition may be partially applied only to regions near the left start portion 11, right start portion 12, left end portion O1, and right end portion O2 of the ingrown fingernail or toenail N.

The above case that the adhesive composition 20 is applied at each corner may be applied when the symptoms of ingrown fingernail or toenail N are not significantly severe.

As described above, one aspect of the present disclosure has been developed and disclosed with respect to the method for correcting the ingrown fingernail or toenail, in which the adhesive composition is applied and the ultraviolet and visible rays are irradiated on the ingrown fingernail or toenail to curve both side portions of the ingrown fingernail or toenail upward by a force of the adhesive composition upon being upwardly curved and cured, thereby outwardly protruding both side end portions penetrating into an inner side of a skin so as to correct the ingrown fingernail or toenail, so that additional correction tool is unnecessary, and thus the inconvenience can be minimized and the correction cost can be reduced.

One aspect of the present disclosure has been described with reference to the accompanying drawings, however, those are merely examples among various embodiments including the essentials of the present disclosure, and the purpose is to make for a person having ordinary skill in the art easily carry out the present disclosure, so it is clear that the present disclosure is not limited to the above-described embodiments. Therefore, the scope of the present disclosure may be understood according to the following claims, all technical ideas within the scope of equivalents by modifications, substitutions, replacements, or the like will be construed as falling within the scope of the present disclosure. Further, it is obvious that some of the configurations in the drawings are provided to be exaggerated or reduced than in actuality to more clearly describe the configuration.

### [Description of Reference Numerals]

N: ingrown fingernail or toenail
I1: left start portion
I2: right start portion
O1: left end portion
O2: right end portion
10: protective base composition
10': protective tape
20: adhesive composition
30: protective coating composition
30': coating tape
50: ultraviolet and visible ray lamp

## Claims

1. A method of correcting an ingrown fingernail or toenail, the method comprising:
applying an adhesive composition on a top portion of an ingrown fingernail or toenail (S10); and
irradiating ultraviolet and visible rays on the adhesive composition 20 applied in the adhesive composition application step S10 to allow both side end portions penetrating into an inner side of a skin of the ingrown fingernail or toenail N to protrude outward (S20).

2. The method of claim 1, further comprising:
attaching a protective base composition 10 or protective tape 10' on the ingrown fingernail or toenail N, before performing step S10 (S5).

3. The method of claim 1, further comprising:
attaching a protective coating composition 30 or coating tape 30' on a top portion of the adhesive composition 20, after performing the step S20 (S25).

4. The method of claim 1, wherein steps S10 to S20 are repeated 1 to 5 times.

5. The method of claim 1, wherein a surface of the adhesive composition 20 of step S10 is upwardly curved and cured when irradiated with ultraviolet or visible rays.
